# EUROPÄISCHE PATENTANMELDUNG

(11) **EP 0 890 337 A2**
(43) Veröffentlichungstag der Anmeldung: **13.01.1999**
(21) Anmeldenummer: 98109205.9
(22) Anmeldetag: 20.05.1998
(51) Int. Cl.: A61B 1/12, A61L 2/00

(54) **Vorrichtung zum Reinigen, Sterilisieren, Transportieren und Lagern von medizinischen Geräten, insbesondere von Endoskopen**

(30) Priorität: 22.05.1997 DE 19721538
(71) Anmelder: BHT Hygiene Technik GmbH, 86316 Friedberg/Derching (DE)
(72) Erfinder: Biermaier, Hans, 86316 Friedberg/Derching (DE)
(74) Vertreter: von Bülow, Tam, Dr.

(57) **Zusammenfassung**

Die Vorrichtung zum Reinigen, Sterilisieren, Transportieren und Lagern von medizinischen Geräten hat einen Reinigungs- und Transportbehälter (1) zur Aufnahme der Geräte. Dieser Behälter ist in eine Reinigungsmaschine einschiebbar und an ein Rohrleitungssystem der Maschine kuppelbar. In der Reinigungsmaschine ist eine Aufbereitungseinrichtung (40) für ein Sterilisationsmittel angeordnet, die durch eine zentrale Steuereinrichtung (33) der Maschine betätigbar ist und in der Maschine selbst flüssiges, gasförmiges und/oder aerosolförmiges Sterilisationsmittel aufbereitet. Es wird ein geschlossenes System geschaffen, in welchem der zu reinigende Gegenstand von seiner Benutzung bis zur nächsten Wiederverwendung hermetisch abgeschlossen ist und in dem er allen erforderlichen Behandlungsschritten und insbesondere auch einer vollständigen Sterilisation unterworfen wird.

## Beschreibung

Die Erfindung bezieht sich auf eine Vorrichtung zum Reinigen, Sterilisieren, Transportieren und Lagern von medizinischen Geräten, insbesondere von Endoskopen.

Um minimal-invasives Instrumentarium und Endoskope nach Gebrauch wieder verwendbar zu machen, ist es notwendig, diese Gerätschaften in allen Teilen, also auch in den inneren Leitungsführungen, Kanälen und Ventilanschlüssen rückstandsfrei zu reinigen, zu trocknen und keimfrei zu machen. Die so aufbereiteten Geräte müssen dann bis zum nächsten Gebrauch keimfrei gelagert werden und auch beim Transport bis hin zum Patienten vor Kontamination geschützt werden.

Zu diesem Zweck schlägt die DE 38 19 257 C1 und die parallele US 5,288,467 eine Reinigungs- und Desinfektionsvorrichtung für medizinische Geräte vor, die einen in den Waschraum einer Reinigungsmaschine einsetzbaren und aus diesem entnehmbaren Transport- und Reinigungsbehälter zur Aufnahme der zu reinigenden Gegenstände besitzt. Dieser Behälter hat eine dicht verschließbare Öffnung zum Beschicken und Entnehmen der Gegenstände, mindestens einen Einlaß zur Zufuhr von Reinigungs- und Desinfektionsmitteln sowie von Trocknungsluft und mindestens einen Auslaß zur Abfuhr dieser Substanzen. Die Einlässe und Auslässe sind mit Rückschlagklappen oder Ventilen ausgestattet, wobei diese Klappen oder Ventile normalerweise geschlossen und nur durch ein unter Druck stehendes Fluid öffenbar sind. Wird der Transport- und Reinigungsbehälter in die Maschine eingeschoben, so werden die Einlässe und Auslässe mit entsprechenden Leitungen der Reinigungsmaschine gekuppelt. Wird der Transport- und Reinigungsbehälter aus der Maschine entnommen, so werden die Einlässe und Auslässe automatisch geschlossen und die Geräte können in dem Behälter aufbewahrt und transportiert werden.

Es hat sich aber herausgestellt, daß die in einer solchen Vorrichtung gereinigten und desinfizierten medizinischen Geräte, insbesondere Endoskope nicht hygienisch einwandfrei gereinigt sind, so daß heute in vielen Ländern gefordert wird, daß die Geräte anschließend sterilisiert werden. Der Begriff "Sterilisieren" ist allerdings in einzelnen Ländern unterschiedlich definiert. Die US-amerikanischen Vorschriften verlangen beispielsweise lediglich eine Keimreduktionsrate von 10⁶, was bedeutet, daß von einer Million Keimen nur noch einer überleben darf. Diese Definition ist in den meisten europäischen Staaten nicht anerkannt und entspricht dort dem Begriff der "Desinfektion". In Deutschland ist dagegen "Sterilisieren" so definiert, daß bei einer Million Sterilisationsvorgängen nur in einem Fall noch ein oder mehrere Keime überleben dürfen. Bei allen übrigen Vorgängen darf dagegen kein einziger Keim überleben.

Zum Sterilisieren der gereinigten und ggf. auch desinfizierten Geräte werden diese bisher der Reinigungsmaschine entnommen und in einen Gassterilisator bzw. einen sog. Autoklaven eingelegt, wo sie mit Sterilisationsmittel behandelt werden. Anerkannte Sterilisationsmittel sind beispielsweise Peressigsäure oder Wasserstoffperoxid. Beide Stoffe sind allerdings relativ instabil und verlieren schon nach kurzer Zeit von ca. 1 bis 2 Stunden ihre Wirkung. Deshalb werden sie normalerweise kurz vor ihrem Einsatz mit einer zweiten Komponente, die Inhibitoren enthält, gemischt. Solche Apparaturen zum Sterilisieren sind beispielsweise aus der US 4,731,222 ,der EP 0 424 127 A2 oder der DE 31 17 574 C2 bekannt.

Weiterer Stand der Technik, der sich mit dem Reinigen medizinischer Instrumente beschäftigt, ist in folgenden Druckschriften zu finden:

Die DE 33 27 466 C2 beschreibt ein Verfahren zum Reinigen und Desinfizieren von medizinischen Geräten, bei dem die Geräte zuerst mit einem Reinigungsmittel behandelt werden, anschließend ein Desinfektionsmittel zusätzlich in die Trägerflüssigkeit eingegeben wird und die Gebrauchsgegenstände abschließend gespült werden, wobei als Desinfektionsmittel eine Mischung aus Glutardialdehyd und/oder Bernsteinsäure-Dialdehyd mit einem Salizylat und einem Polyethylenglykol eingesetzt werden.

Die DE 34 13 386 A1 beschreibt eine Spülmaschine zum Reinigen, Desinfizieren und Trocknen von medizinischem Zubehör. Die zu reinigenden Geräte sind auf einem Traggestell mit Laufrollen und Stützfüßen angeordnet, wobei dieser Wagen in die Spülmaschine einschiebbar ist und Rohre für Spülflüssigkeit und Trocknungsluft aufweist. Die Rohre sind mit Anschlüssen für Spülflüssigkeit und Trocknungsluft der Maschine kuppelbar.

Die DE 37 12 701 C2 beschreibt eine Endoskop-Wasch- und Desinfektionsanlage mit einem Waschraum zur Aufnahme eines Endoskopes und zur wahlweisen Aufnahme einer Waschflüssigkeit, einer Desinfektionsflüssigkeit oder einer Spülflüssigkeit und schlägt vor, die Temperatur der Desinfektionsflüssigkeit zu regeln.

Die DE 196 07 530 A1 beschreibt ein Verfahren zum Reinigen medizinischer Instrumente unter Anwendung eines enzymatischen Reinigers, wobei dieser auf wenigstens 70°C zur Durchführung einer Thermodesinfektion aufgeheizt wird.

Die DE 44 45 434 A1 zeigt eine Spülvorrichtung zum Reinigen von Endoskopen mit einem Boiler für die Zufuhr erhitzter Reinigungs- oder Nachspülflüssigkeit. Zur Vermeidung einer Rekontamination der Zuleitungen ist dort eine zusätzliche Reinigungsvorrichtung für diese Zuleitungen vorgeschlagen, die Reinigungs- oder Desinfektionsflüssigkeit durch die Zuleitungen strömen läßt.

Die DE-AS 27 31 362 zeigt eine Waschmaschine zum Reinigen, Desinfizieren und Trocknen von medizinischem Zubehör, wie Atemmasken, Faltenschläuche und Atembeutel, bei der dieses Zubehör an einem herausnehmbaren Rahmen frei in Magazinen aufgehängt ist und in vertikaler Richtung in Schüttelbewegungen versetzt wird.

Die DE 40 03 987 C2 beschreibt eine Spülvorrichtung zum Reinigen von Endoskopen, bei dem die Endoskope mit einer thermisch desinfizierten Nachspülflüssigkeit nachgespült werden. Das Erwärmen der Nachspülflüssigkeit erfolgt mittels eines Wärmetauschers, der die Nachspülflüssigkeit nach ihrer Desinfektion wieder abkühlt, bevor sie zu den Endoskopen gelangt und dabei die rückgewonnene Wärme zum Aufheizen einer Vorspülflüssigkeit verwendet.

Die DE 32 08 349 A1 zeigt eine Anlage zum nacheinander Reinigen, Desinfizieren und Trocknen von Einrichtungsgegenständen beliebiger Art in einer gegenüber der Atmosphäre dicht verschließbaren Kammer. Die Gegenstände sind innerhalb der Kammer in einer drehbaren Aufnahmeinrichtung aufgenommen, die Horizontal- und Vertikalbewegungen durchführen kann.

Die DE 34 08 752 A1 beschreibt eine Anordnung zur Behandlung einer Anästhesieausrüstung mit einer Kassette, die in ein Waschdesinfektionsgerät eingeschoben wird und nach Reinigung und Desinfektion in einen Trockenschrank gehängt wird. Die Kassette dient anschließend auch als Lager bis zur nächsten Benutzung.

Die DE 34 30 631 A1 zeigt eine Vorrichtung zur Reinigung und Desinfektion von Endoskopen, die in dem Schrank aufgehängt werden. Als Reinigungsmittel wird eine Flüssigkeit verwendet, die über eine an Spannung liegende, nach dem Prinzip der elektrolytischen Dissoziation arbeitenden elektrolytischen Zelle umgewälzt wird.

Die DE 37 12 701 A1 beschreibt eine Endoskop-Wasch- und Desinfektionsmaschine, bei der die Temperatur der Desinfektionsflüssigkeit gemessen und geregelt wird, wobei ein ggf. erforderliches Abkühlen durch Zusatz von kaltem Wasser erfolgt.

Die DE 39 35 621 C2 zeigt eine Durchlaufwaschmaschine für Wäsche mit einer Vorwaschzone, einer Klarwaschzone und einer Spülzone, wobei die Flotte aus der Klarwasch- und/oder der Spülzone Reste von Peressigsäure enthalten kann und zum Vorwaschen verwendet wird, wobei in die zum Vorwaschen bestimmte Flotte noch ein Gas eingeleitet wird, welche eine Verbindung mit Aktivsauerstoff eingeht.

Die DE 38 16 734 A1 und die EP 0 342 499 A2 beschreiben ein Verfahren zum Reinigen von Endoskopen mit einer speziellen Reinigungs- und Desinfektionsmittellösung, die ein schaumarmes, nicht ionogenes Tensid, ein proteolytisches Enzym, einen Komplexbildner, einen Aldehyd aus der Formaldehyd und aliphatischen Dialdehyden mit zwei bis acht Kohlenstoffatomen bestehenden Gruppe und ggf. weitere übliche Reinigungs- und Desinfektionsbestandteile enthält und einen pH-Wert von 6 bis 8 besitzt. Diese Lösung wird auf eine Temperatur von 55 bis 65° erhitzt.

Die DE 37 10 517 C2 beschreibt eine Vorrichtung zum Reinigen von Endoskopen, bei der Reinigungsflüssigkeit unter Druck in einen Arbeitskanal des Endoskopes injiziert wird und zusätzlich eine Strömung von Reinigungsflüssigkeit längs der Außenwand des Endoskopes in Richtung zu seinem distalen Ende fließt und dort durch Strömungsabriß einen Unterdruck erzeugt, der eine Saugwirkung auf die durch den Arbeitskanal fließende Reinigungsflüssigkeit ausübt.

Die DE 296 19 272 U1 zeigt schließlich eine Vorrichtung zum Reinigen und Desinfizieren von Endoskopen mit zwei übereinander angeordneten Druckkammern für die Aufnahme von Endoskopen.

Grundsätzliche Probleme bei diesem Stand der Technik liegen darin, daß entweder die medizinischen Geräte nicht wirklich steril sind oder nach dem Reinigen entnommen und in einen Sterilisator eingelegt werden müssen, was arbeitsaufwendig ist oder schließlich, daß sie nach dem Sterilisieren aufgrund Lagerung und Transport schon wieder kontaminiert werden, bevor sie zum Patienten gelangen.

Aufgabe der Erfindung ist es daher, eine Vorrichtung zu schaffen, die bei einfacher Handhabung ermöglicht, verschmutzte medizinische Geräte vollständig zu sterilisieren und bis unmittelbar vor dem Einsatz am Patienten steril zu halten.

Diese Aufgabe wird durch die im Patentanspruch 1 angegebenen Merkmale gelöst. Vorteilhafte Ausgestaltungen und Weiterbildungen der Erfindung sind den Unteransprüchen zu entnehmen.

Das Grundprinzip der Erfindung besteht darin, ein geschlossenes System vorzusehen, in welches der kontaminierte Gegenstand unmittelbar nach dem Einsatz am Patienten gelangt. Der Gegenstand wird dann transportiert, gereinigt, ggf. desinfiziert, sterilisiert, gespült und getrocknet sowie weiterhin auch transportiert und aufbewahrt, ohne dieses geschlossene System zu verlassen. Ein Umsetzen von einer Reinigungsmaschine in eine Sterilisationseinrichtung entfällt. In dieser gesamten Kette besteht keine Gefahr, daß der Gegenstand nach der Sterilisation rekontaminiert wird, noch daß er selbst vor seiner Reinigung die Umgebung kontaminiert.

Die Vorrichtung nach der Erfindung hat zwei Hauptkomponenten, nämlich einen Behälter zur Aufnahme der Gegenstände, der als Transportmittel, Reinigungs- und Sterilisationsbehälter sowie auch als steriles Lagerungsmittel dient. Die zweite Komponente ist eine einzige Maschine, in die dieser Behälter eingeschoben wird und die alle Arbeitsschritte, wie Reinigen, Spülen, Desinfizieren, Sterilisieren, Nachspülen und Trocknen durchführt. Insbesondere erfolgt in dieser Maschine auch das komplette Herrichten bzw. Aufbereiten des Sterilisationsmittels, einschließlich, falls erforderlich, das Mischen der Komponenten des Sterilisationsmittels und dessen Zufuhr zu den zu sterilisierenden Gegenständen in der gewünschten Form, beispielsweise in Gasform, in Form von Aerosolen oder in Form von Flüssigkeit.

Alle in der Maschine durchgeführten Arbeitsschritte werden von einer einheitlichen Programmsteuerung initiiert und überwacht und können entweder vom Benutzer ausgewählt oder von den zu reinigenden Gegenständen selbst abgerufen werden. Im letzt genannten Fall sind in der Maschine Leseeinrichtungen angeordnet, die beispielsweise über einen Strichcode, einem Transponder, eine magnetische Codierung oder eine Bilderkennung den oder die zu behandelnden Gegenstände identifizieren und aus dieser Information den entsprechend vorprogrammierten Behandlungsablauf auswählen.

Das Aufbereiten des Sterilisationsmittels in der Maschine erfolgt nach einer Variante der Erfindung in einer Ultraschall-Vernebelungskammer, die in ein Bypass-System der Maschine integriert ist. Das Sterilisationsmittel wird dort zu Aerosolen aufbereitet und durch ein Gebläse oder einen sonstigen Drucklufterzeuger an die zu sterilisierenden Flächen geführt. Das Sterilisationsmittel ist damit auch in einem geschlossenen System geführt, wodurch gewährleistet ist, daß toxische Gase nicht austreten können.

Vorzugsweise erfolgt die Zufuhr des aufbereiteten Sterilisationsmittels impulsweise. Zu diesem Zweck ist in der Zufuhrleitung für die Druckluft ein steuerbares Ventil eingefügt, das durch periodisches Öffnen und Schließen für Druckimpulse sorgt.

Die Aufbereitung des Sterilisationsmittels erfolgt chargenweise. Je nach ausgewähltem Sterilisationsprogramm wird über Dosierpumpen oder sonstige Dosiereinrichtungen eine exakt auf die jeweilige Charge abgestimmte Menge an Sterilisationsmittel bzw. an zu mischenden Komponenten desselben in einer Aufbereitungskammer, die auch die genannte Ultraschall-Vernebelungskammer sein kann, zugeführt. Dies erfolgt unmittelbar vor dem Einsatz, so daß auch instabile Lösungen von Sterilisationsmitteln eingesetzt werden können.

Nach einer Variante der Erfindung ist die Aufbereitungseinrichtung für die Sterilisationsmittel unmittelbar in dem Leitungssystem von einer Trocknungslufterzeugungseinrichtung zum Bypass-System der Maschine angeordnet. Nach einer anderen Variante der Erfindung ist diese Einrichtung dagegen separat von der Trocknungsluftaufbereitungseinrichtung an den Bypass der Maschine angeschlossen, wobei dann ein eigenes Absperrorgan für die Aufbereitungseinrichtung vorgesehen ist.

Vorzugsweise sind der Druck oder die Strömungsgeschwindigkeit, mit dem bzw. der das Sterilisationsmittel zugeführt wird, geregelt. Zu diesem Zweck ist in der entsprechenden Zufuhrleitung ein entsprechender Sensor für den Druck oder die Strömungsgeschwindigkeit angeordnet. Aus den Meßwerten dieses Sensors läßt sich dann auch das Volumen des zugeführten Sterilisationsmittels bestimmen.

Um während der Sterilisation eine Kondensation des Sterilisationsmittels zu verhindern, sind die entsprechenden Zuleitungen mit Heizeinrichtungen versehen, die vorzugsweise an der Außenfläche angebrachte elektrische Heizeinrichtungen, wie Widerstandsheizungen oder Widerstandsfolien sind.

Vorzugsweise ist in der Aufbereitungseinrichtung ein Sensor angeordnet, der den Flüssigkeitspegel überwacht und an die zentrale Steuerung meldet. Diese Daten werden in der Prozesssteuerung gespeichert und können als Nachweisprotokoll für eine einwandfreie Sterilisation auch auf einen externen Drucker ausgegeben werden.

Nach einer Weiterbildung der Erfindung besitzt die Vorrichtung eine Aufnahmekammer für Spezialgefäße, die die Komponenten der Sterilisationsflüssigkeit enthalten. Form und Ausstattung dieser Aufnahmekammer sind an diese Spezialgefäße angepaßt, womit ausgeschlossen ist, daß falsche Flaschen mit falschen Flüssigkeiten angeschlossen werden können. Dadurch ist ausgeschlossen, daß der Benutzer ungeeignete Reagenzien einsetzt.

Nach einer Variante der Erfindung wird die Sterilisationsflüssigkeit durch Schwerkraft der Aufbereitungseinrichtung zugeführt. Zur Dosierung sind Ventile vorgesehen. Hierdurch werden zusätzliche Förderpumpen vermieden. Die Menge der der Aufbereitungseinrichtung zugeführten Sterilisationsflüssigkeit bzw. deren Komponenten kann einerseits über die Öffnungs- und Schließzeiten der entsprechenden Ventile ermittelt werden sowie über den erwähnten Flüssigkeitspegelsensor in der Aufbereitungseinrichtung und schließlich auch dadurch, daß die entsprechenden Mengen durch einen Strömungssensor in den entsprechenden Leitungen bestimmt werden, womit insbesondere bei der Verwendung eines aus mehreren Komponenten gemischten Sterilisationsmittels sichergestellt ist, daß die einzelnen Komponenten richtig dosiert sind.

Kurz zusammengefaßt ist es mit der Vorrichtung nach der Erfindung möglich, in einem geschlossenen System medizinische Geräte zu reinigen, zu desinfizieren, zu sterilisieren, zu trocknen und diese Geräte in einem geschlossenen System auch zu lagern und zu transportieren.

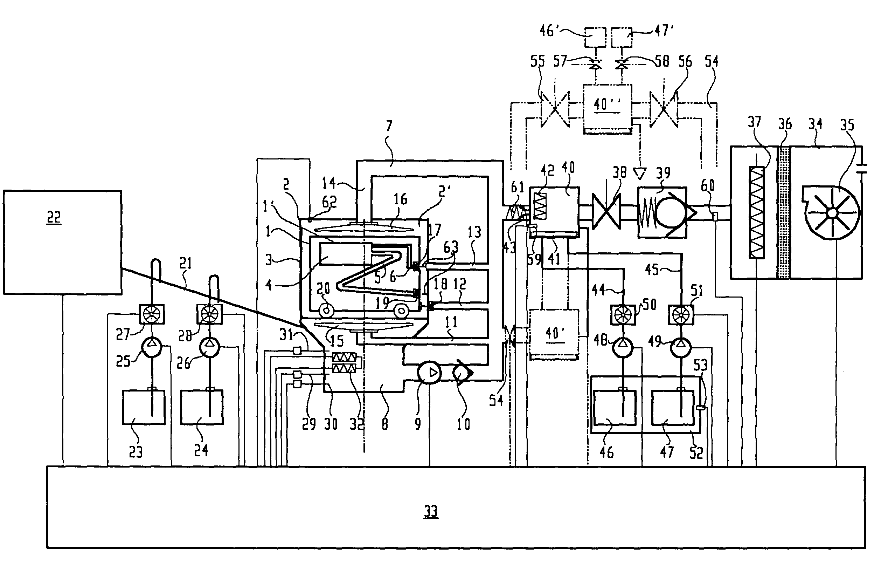

Im folgenden wird die Erfindung anhand eines Ausführungsbeispieles im Zusammenhang mit der Zeichnung ausführlicher erläutert.

Die einzige Figur zeigt eine Prinzipskizze der Vorrichtung nach der Erfindung.

Die Vorrichtung der einzigen Figur zeigt einen Reinigungs- und Transportbehälter 1, der in einen Waschraum 2' einer Reinigungsmaschine 2 durch eine Tür 3 hindurch einschiebbar ist. Der Reinigungs- und Transportbehälter 1 hat eine durch eine Klappe 1' dicht verschließbare Druckkammer 4 zur Aufnahme des zu reinigenden Gegenstandes, wie z.B. eines Endoskops. Der flexible Teil des Endoskops ist in einem an die Druckkammer 4 angeschlossenen Rohr 5 eingeführt. Die Druckkammer ist über eine Leitung 6 mit einem Bypass-System 7 verbunden. Das Bypass-System 7 sorgt für die Umwälzung der Behandlungsflüssigkeiten und ist an einen Sumpf 8, der sich unterhalb des Waschraumes 2' befindet, angeschlossen. Mittels einer Umwälzpumpe 9 wird die im Sumpf 8 befindliche Flüssigkeit in das Bypass-System 7 gepumpt und gelangt über ein Rückschlagventil 10 zu Abzweigleitungen 11 bis 14. Die Abzweigleitung 11 ist mit einem unteren Düsendreharm 15 und die Abzweigleitung 14 mit einem oberen Düsendreharm 16 verbunden. Die Abzweigleitung 13 ist über eine automatische Kupplung 17 mit der Leitung 6 und dann mit der Druckkammer 4 verbunden. Die Abzweigleitung 12 ist ebenfalls über eine automatische Kupplung 18 mit weiteren, hier nicht dargestellten Leitungen des Reinigungs- und Transportbehälters 1 verbunden, beispielsweise mit Aufsteckdüsen oder sonstigen Sprühdüsen. Schließlich ist das Ende des Rohrs 5 mit einem automatischen Absperrventil 19 verschließbar, das sich beim vollständigen Einfahren des Reinigungs- und Transportbehälters 1 in den Waschraum 2' automatisch öffnet und einen Abfluß der Behandlungsflüssigkeiten aus dem Rohr 5 zum Waschraum 2' und damit zum Sumpf 8 der Maschine 2 gestattet. Wird der Reinigungs- und Transportbehälter 1 aus dem Waschraum 2' herausgenommen, so werden die Kupplungen und Ventile 17-19 automatisch geschlossen.

Der Reinigungs- und Transportbehälter 1 hat mehrere Räder 20, um den Transport zu erleichtern. Nebenbei sei bemerkt, daß der hier verwendete Begriff "Reinigungs- und Transportbehälter" nicht einschränkend zu verstehen ist, sondern insbesondere auch die Funktionen Sterilisieren und Aufbewahrung der medizinischen Gegenstände umfaßt.

Die Zufuhr von Reinigungsflüssigkeit erfolgt über eine Rohrleitung 21, die an ein Wasseraufbereitungssystem 22 angeschlossen ist. Weiter sind an diese Rohrleitung 21 Behälter 23 und 24 für Reinigungs- und/oder Desinfektionsflüssigkeit angeschlossen, wobei die entsprechende Flüssigkeit über eine Pumpe 25, 26 sowie einen Durchflußmesser 27 bzw. 28 an die Rohrleitung 21 angeschlossen ist. Ein Ende der Rohrleitung 21 mündet in den Sumpf 8 der Maschine 2. Das Wasseraufbereitungssystem 22 ist im Prinzip bekannt, beispielsweise aus der DE 40 03 987 C2, auf deren Offenbarungsgehalt Bezug genommen wird. Im Sumpf der Maschine sind zwei Niveauschalter 29 und 30 angeordnet, die den Flüssigkeitspegel im Sumpf messen. Weiterhin ist mindestens ein Temperaturfühler 31 vorhanden, der die Temperatur der im Sumpf befindlichen Flüssigkeit mißt sowie schließlich eine Heizeinrichtung 32 zum Aufheizen der von der Pumpe 9 im Bypass-System 7 umgewälzten Flüssigkeit.

Alle beschriebenen Sensoren, Pumpen, Heizeinrichtungen, sofern vorhanden auch Dosiereinrichtungen sowie die nachfolgend noch zu beschreibenden Ventile sind an eine elektrische oder elektronische Steuereinrichtung 33 angeschlossen, die alle Vorgänge steuert und überwacht.

An das Bypass-System 7 der Maschine ist weiterhin - wie an sich bekannt - eine Trocknungseinrichtung 34 angeschlossen, die hier ein Gebläse 35, ein Sterilfilter 36 und eine Heizeinrichtung 37 aufweist und über ein ebenfalls von der Programmsteuerung 33 steuerbares Absperrventil 38 sowie ggf. auch noch ein Rückschlagventil 39 heiße sterile Trocknungsluft unter Druck dem Waschraum 2' und dem Reinigungs- und Transportbehälter 1 zuführt.

Mit der bisher beschriebenen Vorrichtung können medizinische Geräte zwar gereinigt, eventuell auch desinfiziert und getrocknet werden. Auch können die so behandelten Geräte in dem Behälter 1 transportiert und gelagert werden. Wie schon eingangs beschrieben, genügt diese Behandlung aber nicht den erhöhten Anforderungen an die Reinheit der Geräte. Vielmehr wird eine vollständige Sterilität verlangt.

Nach der Erfindung ist daher eine an das Bypass-System 7 angeschlossene Aufbereitungseinrichtung 40 für Sterilisationsmittel angeschlossen. Diese Aufbereitungseinrichtung ist beispielsweise eine Ultraschall-Vernebelungskammer, in der ein elektrischer Ultraschall-Schwinger 41 angeordnet ist, der von der zentralen Steuerung 33 angesteuert wird. Alternativ oder ergänzend hierzu kann in dieser Kammer eine Heizeinrichtung 42 vorgesehen sein, die eine thermische Verdampfung des Sterilisationsmittels bewirkt. Auch kann an dem zum Bypass-System 7 führenden Ausgang eine Vernebelungsdüse 43 angeordnet sein. An die Aufbereitungseinrichtung 40 sind hier zwei Zufuhrleitungen 44 und 45 angeschlossen, über die einzelne Komponenten eines Sterilisationsmittels, die erst in der Aufbereitungseinrichtung 40 miteinander vermischt werden, zuführbar sind. Diese beiden Komponenten stammen aus Behältern 46 und 47 und werden von dort mittels Pumpen 48 bzw. 49 in die Aufbereitungseinrichtung 40 gefördert. Zwischen den Pumpen 48 und 49 und der Aufbereitungseinrichtung 40 sind Durchflußmesser 50 bzw. 51 angeschlossen, die an die Steuereinrichtung 33 ein elektrisches Signal liefern, das die durchgeflossene Menge der entsprechenden Komponente des Sterilisationsmittels anzeigt. Die beiden Pumpen 48 und 49 werden von der Steuereinrichtung 33 gesteuert, so daß im Zusammenwirken mit den Durchflußmessern 50 und 51 die Komponenten des Sterilisationsmittels exakt dosiert werden können.

Die beiden Behälter 46 und 47 sind in einer Aufnahmekammer 52 angeordnet, die hinsichtlich Größe, Form und sonstiger Ausstattung an die Behälter 46 und 47 angepaßt ist Damit wird ausgeschlossen, daß falsche Flaschen mit falschen Flüssigkeiten angeschlossen werden. Weiter kann vorgesehen sein, daß in der Aufnahmekammer 52 Leseeinrichtungen 53 angeordnet sind, die beispielsweise über einen Strich-Code die Behälter 46 und 47 identifizieren und an die Steuereinrichtung 33 melden, worauf diese bei falschen oder nicht identifizierbaren Behältern ein Warnsignal ausgibt bzw. bei richtig identifizierten Behältern mittels eines gespeicherten Unterprogrammes Menge und Mischungsverhältnis der Komponenten in den Behältern 46 und 47 bestimmt.

Bei der bisher beschriebenen Variante der Erfindung ist die Aufbereitungseinrichtung 40 direkt im Strömungsweg von der Trocknungseinrichtung 34 zu dem Bypass-System 7 angeordnet, d.h. Trocknungsluft durchströmt die Aufbereitungseinrichtung 40 und fördert damit auch das aufbereitete Sterilisationsmittel. Durch periodisches Öffnen und Schließen des Ventils 38 durch die Steuerung 33 kann das Sterilisationsmittel auch impulsweise gefördert werden, was dessen Wirkung verbessert, da durch stoßartiges Einpressen des Sterilisationsmittels eine möglichst dichte Konzentration an die zu sterilisierenden Flächen bringt und das Sterilisationsmittel auch durch enge Kanäle von Endoskopen gepreßt wird. Solche Druckimpulse können auch durch impulsweises Einschalten des Gebläses 35 erzeugt werden oder auch durch impulsweises Öffnen des Ventiles 38, das an einen Preßluftbehälter angeschlossen ist. Im übrigen kann auch während des nach dem Sterilisieren erfolgenden Trocknungsvorgang die Trocknungsluft impulsweise dem System zugeführt werden, wodurch eine bessere Trocknungswirkung erzielt wird.

Bei einer mit gestrichelten Linien dargestellten Variante der Erfindung ist die Aufbereitungseinrichtung 40' unabhängig von der Trocknungseinrichtung 34 an das Bypass-System 7 angeschlossen. Die Zufuhr der Komponenten erfolgt in gleicher Weise wie bei dem bisher beschriebenen Ausführungsbeispiel aus den Behältern 46 und 47. In einem Rohrleitungsabschnitt zwischen der Aufbereitungseinrichtung 40' und dem Bypass-System 7 befindet sich ein von der Steuereinheit 33 ansteuerbares Absperrventil 54. Es ist aber auch möglich, hier nur ein Rückschlagventil vorzusehen. Die Förderung des aufbereiteten Sterilisationsmittels aus der Aufbereitungseinrichtung 40' zum Bypass-System 7 erfolgt dabei dadurch, daß beim Aufbereiten des Sterilisationsmittels und dessen Vernebeln oder Verdampfen ein ausreichender Druck erzeugt wird, so daß das aufbereitete Sterilisationsmittel in das Bypass-System gelangt. Es ist aber auch möglich, einen separaten Druckerzeuger, wie z.B. ein Gebläse, eine Pumpe, eine Druckluftquelle oder ähnliches vorzusehen.

Nach einer zweiten, ebenfalls gestrichelt dargestellten Variante ist die Aufbereitungseinrichtung 40'' in einer Bypass-Leitung 54 angeordnet, die parallel zur Trocknungsluftstrecke von der Trocknungseinrichtung 34 zum Bypass-System 7 verläuft und das Ventil 38 überbrückt. Zumindest an einer, vorzugsweise aber an beiden Seiten der Aufbereitungseinrichtung 40'' sind elektrisch steuerbare Ventile 55 und 56 vorgesehen, die von der Steuereinheit 33 betätigt werden. Damit kann wahlweise Trocknungsluft von der Trocknungseinrichtung 34 direkt zum Bypass-System 7 geleitet werden, wobei dann das Ventil 38 geöffnet und die Ventile 55 und 56 gesperrt sind oder die Trocknungsluft kann durch die Aufbereitungseinrichtung 40'' geleitet werden, wobei dann das Ventil 38 geschlossen und die Ventile 55 und 56 geöffnet sind, wobei auch hier durch periodisches Öffnen und Schließen der Ventile 55 und/oder 56 des aufbereitete Sterilisationsmittel impulsweise zugeführt werden kann.

Bei dieser Variante ist auch noch dargestellt, daß die Behälter 46' und 47' oberhalb des Aufbereitungseinrichtung 40'' angeordnet sein können, so daß die Zufuhr der Komponenten des Sterilisationsmittels zur Aufbereitungseinrichtung durch Schwerkraft erfolgt. In diesem Falle kann man die Pumpen 48 und 49 einsparen und stattdessen elektrisch steuerbare Ventile 57 und 58 in die Verbindungsleitungen von den Behältern 46', 47' zu der Aufbereitungseinrichtung 40'' anordnen. Statt der Ventile können auch andere Dosiereinrichtungen verwendet werden oder es können Durchflußmesser in die Zufuhrleitungen eingesetzt werden. Bei einer einfacheren Ausführungsform ist es aber auch möglich, allein durch die Öffnungs- und Schließzeiten der Ventile 57 und 58 die Menge der Komponenten der Sterilisationsflüssigkeit zu dosieren. Die Tätigkeit der Aufbereitungseinrichtung wird ebenfalls durch die Steuereinheit 33 gesteuert. Bei allen geschilderten Varianten kann in der Dosiereinrichtung 40 noch mindestens ein Sensor 59 vorhanden sein, der den Flüssigkeitspegel mißt und an die Steuereinheit 33 meldet. Selbstverständlich können auch weitere Kenngrößen, wie Temperatur, Druck, elektrische Leitfähigkeit, pH-Wert oder sonstige Parameter des Sterilisationsmittels durch Sensoren gemessen werden und als Meßprotokoll in der Steurerung 33 gespeichert und später ausgedruckt werden.

In der Zufuhrleitung von der Trocknungseinrichtung 34 zum Bypass-System 7 ist ein Sensor 60 angeordnet, der die für die Eintragung des Sterilisationsmittels verwendete Luft mißt. Es kann sich hier um einen Strömungsmesser oder einen Drucksensor handeln oder beides. Auch diese Meßwerte werden in der Steuerung 33 abgespeichert und ggf. später in einem Protokoll ausgedruckt, womit alle relevanten Prozeßdaten nicht nur überwacht sondern auch dokumentiert werden können.

Selbstverständlich können bei den Varianten der anders angeordneten Aufbereitungseinrichtungen 40' und 40'' entsprechende Sensoren vorhanden sein.

Um ein Kondensieren und damit Niederschlagen des aufbereiteten Sterilisationsmittels an Rohrleitungen oder dem Bypass-System zu verhindern, können weiterhin Heizeinrichtungen 61 vorgesehen sein, die die entsprechenden Rohrleitungen oder Teile bzw. das gesamte Bypass-System 7 während der Sterilisation aufheizen. Vorzugsweise sind diese Heizeinrichtungen 61 elektrische Heizeinrichtungen, wie z.B. Widerstandsheizung oder Widerstandsfolien, die außen an den entsprechenden Zuleitungen, beispielsweise dem Leitungsabschnitt zwischen der Aufbereitungseinrichtung 40 und dem Bypass-System angeordnet sind. Auch der Einsatz dieser Heizeinrichtungen wird von der Steuereinrichtung 33 gesteuert und, falls gewünscht, auch abgespeichert und protokolliert.

Die Reinigungsmaschine 2 weist eine Leseeinrichtung 62 auf, die die in oder auf dem Reinigungs- und Transportbehälter befindlichen Gegenstände identifiziert und an die zentrale Steuerung 33 meldet, welche daraufhin einen vorprogrammierten Behandlungsablauf aufruft. Die Leseeinrichtung 62 kann beispielsweise ein Strichcodeleser sein, wobei dann die zu reinigenden Gegenstände mit einem entsprechenden Strichcode versehen sein müssen. Auch andere Techniken zum Identifizieren von Gegenständen können angewandt werden, wie z.B. eine Bilderkennung, Übertragung von Identifikationsdaten durch Transponder oder ähnliches.

Schließlich ist noch zu erwähnen, daß die am Einlaß und am Auslaß zur Druckkammer 4 befindlichen Absperrorgane 17 und 19 durch im Inneren des Waschraumes 2' angeordnete Betätigungsorgane 63 beim Einschieben des Reinigungs- und Transportbehälters 1 in den Waschraum 2' automatisch geöffnet werden. Sind die Absperrorgane 17 und 19 als Rückschlagklappen ausgebildet, so kann das Betätigungsorgan ein einfacher Stift sein. Ansonsten können hier alle bekannten selbstsperrenden Kupplungen für Rohrleitungssysteme verwendet werden.

## Patentansprüche

1. Vorrichtung zum Reinigen, Sterilisieren, Transportieren und Lagern von medizinischen Geräten, insbesondere von Endoskopen mit
einem Reinigungs- und Transportbehälter (1) zur Aufnahme der Geräte, wobei der Reinigungs- und Transportbehälter (1) eine dicht verschließbare Öffnung (1') zum Beschicken, einen dicht verschließbaren Einlaß (17) für Reinigungs- und Sterilisationsmittel sowie für Trocknungsluft und einen dicht verschließbaren Auslaß (18) aufweist,
mit einer Reinigungsmaschine (2) zur Aufnahme des Reinigungs- und Transportbehälters (1), die Kupplungen zum Anschließen des Einlasses (17) und des Auslasses (18) sowie eine zentrale Steuereinrichtung (33) aufweist,
wobei in der Reinigungsmaschine (2) eine Aufbereitungseinrichtung (40) für ein Sterilisationsmittel angeordnet ist, die durch die Steuereinrichtung (33) betätigbar ist und flüssiges, gasförmiges und/oder aerosolförmiges Sterilisationsmittel aufbereitet, wobei die Aufbereitungseinrichtung (40) mit dem Einlaß (17) verbindbar ist und
wobei Fördermittel (34, 41) vorgesehen sind, die das aufbereitete Sterilisationsmittel in den Reinigungs- und Transportbehälter (1) fördern.

2. Vorrichtung nach Anspruch 1, bei der die Reinigungsmaschine (2) ein Bypass-System (7) aufweist, in welchem die Reinigungsflüssigkeit umgewälzt wird, dadurch gekennzeichnet,
daß die Aufbereitungseinrichtung (40) für das Sterilisationsmittel an das Bypass-System (7) angeschlossen ist.

3. Vorrichtung nach Anspruch 1 oder 2, dadurch gekennzeichnet,
daß die Aufbereitungseinrichtung (40) einen Ultraschall-Zerstäuber (41) aufweist.

4. Vorrichtung nach einem der Ansprüche 1 bis 3, dadurch gekennzeichnet, daß die Aufbereitungseinrichtung (40) eine Heizeinrichtung (42) zum thermischen Verdampfen des Sterilisationsmittels aufweist.

5. Vorrichtung nach einem der Ansprüche 1 bis 4, dadurch gekennzeichnet,
daß die Aufbereitungseinrichtung (40) eine Vernebelungsdüse (43) aufweist.

6. Vorrichtung nach Anspruch 1, dadurch gekennzeichnet,
daß das Fördermittel eine Turbinengebläse (35) ist, das sterile Druckluft in die Aufbereitungseinrichtung (40) fördert.

7. Vorrichtung nach Anspruch 6, dadurch gekennzeichnet,
daß eine Heizeinrichtung (37) zum Erwärmen der von dem Turbinengebläse (35) geförderten Druckluft vorgesehen ist.

8. Vorrichtung nach einem der Ansprüche 1 bis 7, dadurch gekennzeichnet,
daß Mittel (38, 55, 56, 35) vorgesehen sind, die das Sterilisationsmittel stoßweise mit Druckimpulsen den zu sterilisierenden Gegenständen zuführen.

9. Vorrichtung nach Anspruch 8, gekennzeichnet durch mindestens einen Sensor (60), der den Druck und/oder die Strömungsgeschwindigkeit mit dem bzw. der das Sterilisationsmittel zu den zu sterilisierenden Gegenständen gefördert wird, mißt und daß die entsprechenden Meßwerte in der Steuereinheit (33) gespeichert werden.

10. Vorrichtung nach einem der Ansprüche 1 bis 9, dadurch gekennzeichnet,
daß die Aufbereitungseinrichtung (40) eine unmittelbar in ein Rohrleitungssystem der Reinigungsmaschine (2) integrierte Kammer aufweist.

11. Vorrichtung nach einem der Ansprüche 1 bis 10, dadurch gekennzeichnet,
daß eine oder mehrere Dosierpumpen (48, 49, 50, 51) vorgesehen sind, die von der Steuereinheit (33) gesteuert werden, derart, daß die benötigte Menge des Sterilisationsmittels chargenweise zudosiert wird.

12. Vorrichtung nach einem der Ansprüche 1 bis 10, dadurch gekennzeichnet,
daß Behälter (46', 47') für das Sterilisationsmittel bzw. dessen Komponenten oberhalb der Aufbereitungseinrichtung (40'') angeordnet sind, so daß die Zufuhr in die Aufbereitungseinrichtung (40'') durch Schwerkraft erfolgt.

13. Vorrichtung nach einem der Ansprüche 1 bis 12, dadurch gekennzeichnet,
daß an einem von dem aufbereiteten Sterilisationsmittel durchströmten Rohrleitungssystem eine weitere Heizeinrichtung (61), vorzugsweise in Form einer Widerstandsheizung oder einer Widerstandsfolie angebracht ist zur Verhinderung einer Kondensation des Sterilisationsmittels.

14. Vorrichtung nach einem der Ansprüche 1 bis 13, dadurch gekennzeichnet,
daß die Aufbereitungseinrichtung (40) einen Sensor (59) aufweist, der den Flüssigkeitspegel der Sterilisationsflüssigkeit überwacht und an die Steuereinheit (33) meldet.

15. Vorrichtung nach einem der Ansprüche 1 bis 14, dadurch gekennzeichnet,
daß in der Reinigungsmaschine (2) eine Aufnahmekammer (52) zur Aufnahme mindestens eines Behälters (46, 47) für Sterilisationsflüssigkeit vorgesehen ist und daß die Aufnahmekammer hinsichtlich Form, Größe und sonstiger Ausstattung an den mindestens einen Behälter (46, 47) angepaßt ist derart, daß ein Anschließen von falschen Behältern ausgeschlossen ist.

16. Vorrichtung nach einem der Ansprüche 1 bis 15, dadurch gekennzeichnet,
daß die Aufbereitungseinrichtung (40) im Strömungsweg von einem Trocknungslufterzeuger (32, 34, 35, 36) zum Bypass-System (7) der Reinigungsmaschine (2) angeordnet ist.

17. Vorrichtung nach einem der Ansprüche 1 bis 15, dadurch gekennzeichnet,
daß die Aufbereitungseinrichtung (40'') in einem Bypass (54) zu einem Strömungsweg von einem Trocknungslufterzeuger (32, 34, 35, 36) zum Bypass-System (7) der Reinigungsmaschine (2) angeordnet ist und daß von der Steuereinheit (33) steuerbare Ventile (38, 55, 56) vorgesehen sind, die wahlweise diesen Bypass (54) und den Strömungsweg vom Trocknungslufterzeuger öffnen oder schließen.

18. Vorrichtung nach einem der Ansprüche 1 bis 15, dadurch gekennzeichnet,
daß die Aufbereitungseinrichtung (40') unmittelbar an das Bypass-System (7) der Reinigungsmaschine (2) angeschlossen ist.

19. Vorrichtung nach einem der Ansprüche 1 bis 18, dadurch gekennzeichnet,
daß die zu sterilisierenden Gegenstände eine maschinell lesbare Identifizierung aufweisen und daß eine an die Steuereinheit (33) angeschlossene Leseeinrichtung (62) vorgesehen ist, die die zu reinigenden Gegenstände identifiziert, worauf die Steuerung (33) selbsttätig einen vorprogrammierten Behandlungsablauf auswählt.

20. Vorrichtung nach einem der Ansprüche 1 bis 19, dadurch gekennzeichnet,
daß der Einlaß (17) und der Auslaß (18) ein steril abschließbares Absperrorgan aufweisen und daß im Waschraum (2') der Reinigungsmaschine (2) Betätigungsorgane (63) so angeordnet sind, daß die Absperrorgane beim Herausziehen des Reinigungs- und Transportbehälters aus dem Waschraum (2') automatisch geschlossen und beim Einschieben automatisch geöffnet werden.
